# EUROPEAN PATENT SPECIFICATION

(11) **EP 3 457 130 B1**
(45) Date of publication and mention of the grant of the patent: **30.03.2022**
(21) Application number: 17191948.3
(22) Date of filing: 19.09.2017
(51) Int. Cl.: G01N 33/28

(54) **APPARATUS AND METHOD FOR MONITORING A CONDITION OF METALWORKING FLUID OF A METALWORKING FLUID CIRCUIT OF A METALWORKING MACHINE**
VORRICHTUNG UND VERFAHREN ZUR ÜBERWACHUNG EINES ZUSTANDS EINER METALLBEARBEITUNGSFLÜSSIGKEIT EINES METALLBEARBEITUNGSFLÜSSIGKEITSKREISLAUFS EINER METALLBEARBEITUNGSMASCHINE
APPAREIL ET PROCÉDÉ POUR SURVEILLER UN ÉTAT DE FLUIDE DE TRAVAIL DES MÉTAUX D'UN CIRCUIT DE FLUIDE DE TRAVAIL DES MÉTAUX D'UNE MACHINE DE TRAVAIL DES MÉTAUX

(30) Priority: 19.09.2016 BE 201605702
(43) Date of publication of application: 20.03.2019
(73) Proprietor: BVBA Dierickx-Tools, 3111 Wezemaal (BE)
(72) Inventor: Dierickx, Pieter, 3111 Wezemaal (BE); Dierickx, Dirk Guillaume Gilbert, 3111 Wezemaal (BE)
(74) Representative: V.O.

(56) References cited:
- DE-A1-102013 009 370
- US-A- 4 315 421
- US-A1- 2015 064 741
- Henry Hopper: "A dozen ways to measure fluid level and how they work:", Sensors Online, 1 December 2004 (2004-12-01), XP055373566, Retrieved from the Internet: URL:http://www.sensorsmag.com/components/a -dozen-ways-to-measure-fluid-level-and-how -they-work [retrieved on 2017-05-17]

## Description

The invention relates to a metalworking machine, in particular to a metalworking machine having an individual metalworking fluid circuit.

Metalworking machines are typically used for automated or semi-automated working of a metallic workpiece, by means of a working tool such as a cutting tool, a milling tool or a machining tool. Such metalworking machines employ a metalworking fluid to lubricate and cool the interface between the working tool and the workpiece. In addition to cooling and lubrication, the metalworking fluid may carry away the shavings and/or chips of material removed from the workpiece.

Often, a water-based metalworking fluid is used comprising a water component, a lubricant fluid component and, preferably, an additive component. The lubricant fluid component may comprise mineral oil, synthetic oil or semi-synthetic oil. In addition, the metalworking fluid may comprise additives, such as corrosion inhibitors, emulsifiers, microbiocides etc. The oil, whether mineral, synthetic or semi-synthetic, is soluble in the water and provides for the lubrication function of the metalworking fluid. The water of the metalworking fluid mainly provides for the cooling function.

A water-based, or aqueous, metalworking fluid is advantageous in use in view of the relative high heat absorption capacity of the fluid, they may be more economical and/or less hazardous than other types of metalworking fluids.

However, a water-base metalworking fluid may be susceptible to evaporation, may provide a medium for the growth of biological contaminants, such as bacteria, mould or other micro-organisms which degenerate the metalworking fluid.

Evaporation, in particular of the water in the water-based fluid, may result in a changed concentration of the fluid between the watercomponent and the lubricant fluid-component. A changed concentration may deteriorate the cooling and/or lubrication capacity of the metalworking fluid. The concentration of the metalworking fluid may also change due to carrying off of the fluid, in particular of the lubricant or an emulsifier, with the parts or chips removed from the workpiece. Also, during use, the volume of the metalworking fluid present in the metalworking fluid circuit may reduce due to the use, carrying off, evaporation etc.

A huge problem with the water-based metalworking fluid is to prevent and/or to control the growth of microbiological organisms in the fluid. Growth of such microbiological organisms may rapidly decrease the quality of the metalworking fluid, which eventually may result that the metalworking fluid is not suitable for further use. Microbiological organisms in the metalworking fluid grow with increasing rapidity, rapidly deteriorating the quality of the metalworking fluid and making it unusable. Then, the metalworking machine, the metalworking fluid circuit etc. needs to be emptied, cleaned and re-filled with clean metalworking fluid. This is a work-intensive job to do, while, additionally, the machine is in downtime, involving significant costs for cleaning, replacement fluid, downtime etc.

Therefore, it is desirable to monitor the quality of the metalworking fluid, preferably to prevent or timely detect growth of microbiological organisms. Thereto, a number of solutions are proposed in the prior art. For example, it is proposed to measure one or more parameters of the metalworking fluid, such as a refractory index and concentration, or pH and conductivity, or concentration and pH, or the conductivity, or pH and dissolved oxygen. A drawback of the proposed prior art devices may be that the sensors to measure the aforementioned parameters are positioned in the metalworking fluid circuit, which makes them vulnerable to damages, contaminants etc. As such, the sensors are continuously exposed to the metalworking fluid which may negatively impact the lifetime of the sensors. Also, maintenance and/or replacement of the sensor may be difficult and/or costly. Another drawback of the proposed prior art devices may be that the metalworking fluid circuit is a circuit on which multiple metalworking machines are connected. So, multiple metalworking machines are serviced by the same metalworking fluid. The risk on deteriorating of the metalworking fluid, e.g. due to microorganism growth, may be relatively high and/or the costs of replacement of deteriorated fluid by replacement fluid are relatively high as well. Also, it may be difficult to control a condition of the metalworking fluid and/or to adapt the composition of the metalworking fluid dependent on the metalworking process. Also, such devices may be complex and it may not be possible to retrofit existing machines with such devices.

Publication DE 102013009370 describes a device for measuring fluid parameters in a fluid pipe using a pair of condenser measurement electrodes. The pair of condenser measurement electrodes is arranged spirally around a central axis of the fluid pipe such that the distance between the electrodes is constant over the length of the spiral winding. The fluid pipe in which the condenser measurement electrodes are arranged, can be connected in a fluid circuit, such that at one end of the fluid pipe, fluid flows in, and at the other end of the fluid pipe, the fluid flows out. During passage of the fluid in the fluid pipe, along the electrodes, a fluid parameter is measured.

It is an object of the invention to provide for a device for monitoring the quality of the metalworking fluid that obviates at least one of the above mentioned drawbacks. In particular, it is an object of the invention to provide for a device that is compact, relatively easy to use and to maintain and that, advantageously, may be suitable for retro-fitting on an existing metalworking machine.

Thereto, the invention provides for a monitoring apparatus for monitoring a condition of an aqueous metalworking fluid comprised of water and a lubricant, of a metalworking fluid circuit of a metalworking machine, comprising a housing having a sample inlet for receiving a sample of metalworking fluid there through from the metalworking fluid circuit; an outlet for discharging the sample of metalworking fluid to the metalworking fluid circuit after measurement; a water connection for connecting to a water feed line; a lubricant connection for connecting to a lubricant feed line; comprising measurement elements arranged in the housing for measuring pre-determined parameters of the sample of metalworking fluid.

By providing such a monitoring apparatus having a housing in which the measurement elements are arranged and in which a sample of the metalworking fluid can be measured, a compact self-contained device can be obtained in which the measurement elements are remote from the metalworking fluid circuit. The measurement elements being remote from the fluid circuit means that the measurement elements are not mounted into the fluid circuit, as in the prior art devices, and as such, are not permanently exposed to the metalworking fluid. Instead, the measurement elements are only exposed to the metalworking fluid during a sample run, so not continuously, but in a discrete fashion.

Advantageously, the housing is provided with an inlet for receiving a sample of the metalworking fluid. Preferably, the inlet is connectable to the metalworking fluid circuit, such that, when the inlet is opened, a sample of the metalworking fluid can pass through. For example, the housing can be provided with a pump unit that can pump a sample from the metalworking fluid from the metalworking fluid circuit to the apparatus. Further, the housing is provided with a water connection for connecting to a water feed line. As such, after each measurement run, the measurement elements can be rinsed with water for cleaning. Thus contamination of the measurement elements may be obviated, and, contrary to the prior art, the measurement elements may not permanently be exposed to the metalworking fluid. The housing is provided with a lubricant connection for connecting to a lubricant feed line. As such, when lubricant is to be added to the metalworking fluid circuit, it can be added via the housing of the monitoring apparatus. For example, a sample can be measured first, e.g. until a desired concentration is obtained, prior to be released in the metalworking fluid circuit. So, a self-contained sample apparatus can be obtained that can monitor pre-determined parameters of the sample of the metalworking fluid, and that allows adjusting of the concentration of the metalworking fluid by means of the water connection and/or the lubricant connection.

Advantageously, the apparatus is provided with at least one measurement chamber. At least one measurement element may be present in, or extend into, the measurement chamber. In another embodiment, all measurement elements may be present in, or extend into, the measurement chamber, or for each measurement element a measurement chamber can be provided, or variants thereof may be possible. By providing such measurement chamber, the sample of metalworking fluid can be supplied to the measurement chamber. Then the measurement run can be done, and after the measurement run, the sample of the metalworking fluid can be discharged into the metalworking fluid circuit and the measurement chamber can be emptied. Preferably, after each measurement run, the measurement chamber and the measurement elements are rinsed with water for cleaning. Thus contamination of the measurement elements may be obviated.

So, contrary to the prior art, there is no continuous measuring of the determined parameters, but a measurement run can be done at specified time intervals. For example a measurement run can be done after a certain event has occurred, such as filling of the circuit with metalworking fluid, or a measurement run can be done at predetermined time intervals, e.g. every hour or every two hours. Also, the time interval may vary depending on the measured parameter values. For example, when deterioration of a parameter value may be detected, the time interval may be shortened to more closely follow up on the monitoring of the condition of the metalworking fluid.

In an advantageous embodiment, a mixer is provided in the measurement chamber. The mixer can be provided to mix the sample of metalworking fluid prior to a measurement run, as to undo some effects that may have occurred during the flow of the sample of fluid from the fluid circuit to the measurement chamber. Also, when water and/or lubricant may have to be added to the fluid circuit, the determined amount of water and/or lubricant is preferably entered into the measurement chamber first, can there be mixed or stirred by the mixer prior to be discharged to the metalworking fluid circuit. So, the added metalworking fluid is already mixed when joining the metalworking fluid circuit, providing for a more optimal mixture with the metalworking fluid already present in the circuit.

The housing of the apparatus is provided with a water connection and a lubricant connection for connecting to a water feed line and a lubricant feed line respectively. The water feed line typically can be a feed line connected to a tap water connection point. The lubricant feed line typically is connected to a container or reservoir containing lubricant. Advantageously, the lubricant, comprising oil, is already provided with the required additives for the metalworking fluid.

By providing the water feed line connection and the lubricant feed line connection onto the apparatus, the apparatus can be more self-contained apparatus, which can be advantageous when water and/or lubricant needs to be supplied, the components can then first be mixed in the apparatus prior to be discharged to the metalworking fluid circuit. Also, this may be advantageous, when the apparatus may be provided with a control unit, that, depending on the measured parameter values, may determine an amount of water and/or an amount of lubricant to be filled. Maintaining a predetermined level of fluid in the circuit and/or a predetermined concentration of the fluid in the circuit may thus be more or fully automated.

Advantageously, the measurement elements are configured to provide the values of the parameters that are measured to a control unit. The control unit may be integrated to the apparatus, i.e. may be arranged in the housing of the apparatus. In another embodiment, the control unit may be remotely located from the apparatus, for example the control unit may be provided on a remote server, or on a mobile communications device, etc. Many variants are possible. By providing such a control unit, the process of monitoring the condition of the metalworking fluid may be more automated, and more information may be provided for example to an operator of such a metalworking machine.

Various parameters can be measured. For example, pH, temperature and/or dissolved oxygen may be measured to give an idea of the micro-organisms growth. When the temperature of the fluid is higher, it is known that micro-organisms may grow more rapidly. Also, the growth of micro-organisms consumes oxygen, so the amount of dissolved oxygen is related to a quantity and/or growth of the micro-organisms. The pH value is then related to the growth of the micro-organisms. Other parameters, such s viscosity, density etc. may be measured as well. Also, the level of the fluid in the metalworking fluid circuit is preferably measured, as there is a minimum amount of fluid required for the operation of the metalworking machine. Further, optical characteristics of the fluid such as the refractory index may be measured, and/or the conductivity may be measured which relate to the concentration of the sampled fluid.

Measuring at least some of these parameters, e.g. pH and refractory index, may provide information about the condition of the metalworking fluid.

Advantageously, the control unit may be configured to receive the values of the measured parameters, and further to determine whether the measured parameter values are within predetermined boundaries. For each parameter, there are predetermined boundaries within which the value of the respective parameter preferably lies. These boundaries may be obtained from external sources, e.g. from a manufacturer of the lubrication fluid setting boundaries for concentration etc., or from experience from a machine operator knowing that the pH preferably is between certain boundaries, etc. This information can be inputted and/or set into the control unit. The control unit may provide an output signal when is determined that a value of a measured parameter is outside the pre-set boundary or boundaries of that respective parameter. This output signal can be a warning signal that can be outputted to an operator of the metalworking machine. For example, the apparatus may comprise a user feedback element that is configured to receive an output signal of the control unit, when a measured parameter value is outside of the predetermined boundaries of the respective parameter. The user feedback element may for example be a warning light, emitting a light signal, probably a flashing light signal, to visually warn a user or an operator, or may be a sound emitter for emitting sound waves to audibly warn a user or an operator. The user feedback element may also be a user interface provided e.g. on the housing of the apparatus on which the information about the respective parameter can be displayed. The output signal of the control unit may also be transmitted to a mobile communication device, or to a control room, or to a remotely located computer etc. Many variants are possible. By providing such an output signal, the user or operator of the metalworking machine is warned that at least one of the measured parameters has a value outside of its preset boundaries.

The operator can then judge whether he may need to take some action and/or what action he might take.

Advantageously, the housing is configured to engage with a metalworking machine, such that, when engaged, the sample inlet is connectable to the metalworking fluid circuit. By engaging the housing to the metalworking machine, no complex connection lines or intermediate components are required, and the sample inlet can be directly connected to the metalworking fluid circuit. Advantageously, engagement elements are provided to the housing for engaging with the metalworking machine. The engagement elements may be holes for receiving fastening elements such as bolts or screws there through, or may be pins to connect with holes in the metalworking machine, etc. Many variants are possible. In an embodiment, the metalworking machine may be provided with seats that are arranged for receiving add-on modules such as a pump module.

Advantageously, the housing of the monitoring apparatus is provided with engagement elements that correspond to a receiving seat of the metalworking machine. As such, the apparatus may easily be fitted to the metalworking machine. In a preferred embodiment, the apparatus is an add-on module for a metalworking machine. A used metalworking machine may thus be easily retrofitted with such an add-on module. Alternatively, new-built metalworking machines can be fitted with such an add-on module, if the customer requires so. The new-built metalworking machine can thus be of a modular design, on which add-on modules, such as the apparatus according to the invention, or any pump module, can be fitted when required. This provides for a large flexibility of the metalworking machine, both for the customer and the manufacturer.

The invention further relates to a method for monitoring a condition of the metalworking fluid in a metalworking fluid circuit of a metalworking machine. The method preferably comprises the steps of sampling an amount of metalworking fluid from the metalworking fluid circuit; measuring at least one predetermined parameter of the sample of metalworking fluid and, after measuring, discharging the sample of metalworking fluid back to the metalworking fluid circuit. By sampling an amount of metalworking fluid, a measurement run can be done on the sample, and contrary to the prior art, there is no continuous measuring and no continuous exposure of the measurement elements to the metalworking fluid. Thereby, the lifetime of the measurement element may be increased. A measurement run can be done at predetermined time intervals, or after an event has taken place. Such an event may be the filling of the metalworking circuit with fluid or supplying additional water and/or lubricant fluid. Also, the time interval between subsequent sample runs may dependent on the results of the previous measurement run. So, when the condition of the metalworking fluid is detected to be decreasing, albeit still within the predetermined boundaries, then the time interval between subsequent sample runs may be shortened to more closely monitor the condition of the metalworking fluid.

Advantageously, it is determined whether a measured parameter value is within a predetermined boundary and an output signal may be outputted when at least one measurement of a parameter is outside of a predetermined boundary.

In another embodiment, the refractory index of the metalworking fluid can be measured by means of a refractometer. The refractory index is related to the concentration of the metalworking fluid. Prior to the first measurement of a newly filled or re-filled metalworking fluid circuit, the refractometer is calibrated. A measurement run with only water is performed, and then a measurement run with the metalworking fluid is done. Based on the refractory indexes of these two measurement runs, a refractory index of the fresh metalworking fluid is determined, serving as a calibration point for further measurements.

The volume in the metalworking fluid circuit preferably is measured by a floater in the tank of the circuit. Preferably, the volume is measured continuously and/or intermittently. The volume of the metalworking fluid in the circuit is likely to decrease during use of the metalworking machine due to evaporation, carrying away with chips and shavings etc. and preferably, the volume is regularly measured. Once the volume of the fluid drops below a certain predetermined boundary, a measurement run to measure the refractory index can be performed. Based on the measured refractory index, the concentration of the fluid available in the circuit can be determined. Further, based on the volume and on the concentration of the metalworking fluid, it can be determined what amount of water and/or what amount of lubricant fluid needs to supplied additionally to refill the circuit until the predetermined volume and/or desired concentration are reached. After refilling the circuit with water and/or lubricant fluid, a measurement run can be done to measure the refractory index and to determine whether the concentration has reached the determined concentration level. Based on this information, it can be determined whether the previously determined amount of water and/or amount of lubricant fluid was sufficient to reach the desired concentration. Such a feedback may be taken into account a next time when re-filling of the circuit is required and the amount of water and/or lubricant fluid have to be determined. So, there may be a learning effect to the method and the control unit may be a learning controller using information from previous measurement runs. This may be advantageous in view of efficient use of, in particular, lubricant fluid, as this may be a relatively expensive component.

The invention further relates to a control unit configured for monitoring a condition of metalworking fluid of a metalworking circuit of a metalworking machine. By providing such a control unit, the monitoring of the condition of the metalworking fluid can be automated, while the control unit can be provided in the apparatus or remote of the apparatus.

The invention further relates to a system comprising the monitoring apparatus and the control unit.

The invention further relates to a method for retro-fitting a metalworking machine having an individual metalworking fluid circuit with the monitoring apparatus.

The disclosure further relates to a retro-fitted metalworking machine and to a newly built metalworking machine having such an apparatus.

The invention further relates to a computer program product for monitoring a condition of a metalworking fluid. The disclosure also relates to a mobile communication device comprising a computer program product.

The disclosure further relates to a kit of a monitoring apparatus and fixating elements.

The present invention will be further elucidated with reference to figures of exemplary embodiments. Corresponding elements are designated with corresponding reference signs.
Figure 1a shows a perspective and schematic view on a metalworking machine including a monitoring apparatus according to a first aspect of the invention;
Figure 1b shows an enlargement of the monitoring apparatus of Figure 1a;
Figure 2 shows a schematic representation of an inside of the monitoring apparatus of Figure 1;
Figure 3 shows a schematic flow diagram of a method for monitoring a condition of metalworking fluid of a metalworking fluid circuit of a metalworking machine.

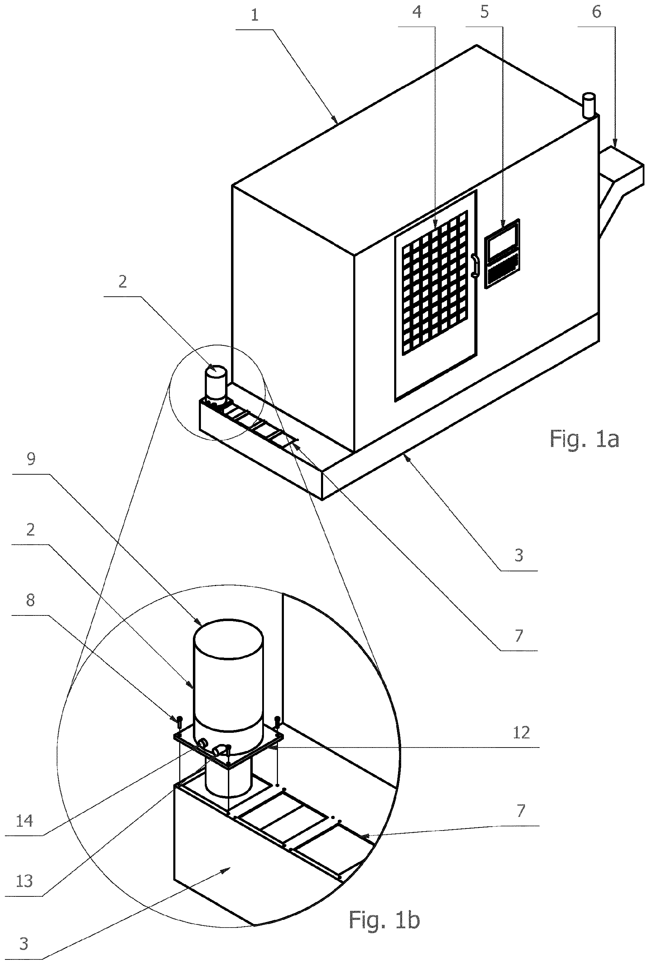

Figure 1a shows a perspective and schematic view on a metalworking machine 1 including a monitoring apparatus 2 for monitoring a condition of an aqueous metalworking fluid of a metalworking fluid circuit 3 of a metalworking machine 1. A metalworking machine 1 can typically be used for automated or semi-automated working of a metallic workpiece, by means of a working tool such as a cutting tool, a milling tool or a machining tool. Such a working tool can be positioned inside the metalworking machine 1, to which access can be provided via a door 4 to introduce the metal piece to be treated into the machine. The working tool, or the entire metalworking machine 1 can be operated from the outside, for example via an interface 5 on the metalworking machine, or via any other remotely located interface. Such metalworking machines 1 generally employ a metalworking fluid to lubricate and cool the interface between the working tool and the workpiece. In addition to cooling and lubrication, the metalworking fluid may carry away the shavings and/or chips of material removed from the workpiece, for example via an residue outlet 6, draining away a mixture of residual metal shavings and a bit of metalworking fluid. The aqueous metalworking fluid in the metalworking fluid circuit 3 is often a water-based metalworking fluid comprising a water component, a lubricant fluid component and, preferably, an additive component. The lubricant fluid component may comprise mineral oil, synthetic oil or semi-synthetic oil. In addition, the metalworking fluid may comprise additives, such as corrosion inhibitors, emulsifiers, microbiocides etc. The oil, whether mineral, synthetic or semi-synthetic, is soluble in the water and provides for the lubrication function of the metalworking fluid. The water of the metalworking fluid mainly provides for the cooling function. The metalworking machine 1 may be provided with one or more receiving seats 7, in which for example a pump can be installed that is configured to pump around the metalworking fluid in the metal working fluid circuit 3 linked to one or more metalworking tool in the metalworking machine 1.

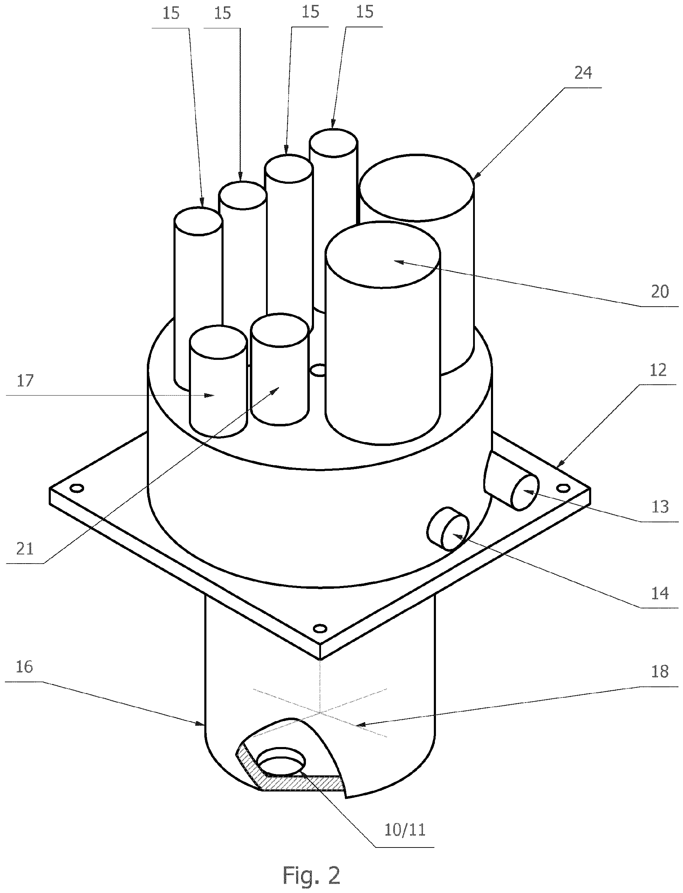

Figure 1b shows an enlargement of the monitoring apparatus of Figure 1a. A monitoring apparatus 2 is provided for monitoring a condition of an aqueous metalworking fluid in a metalworking fluid circuit 3 of a metalworking machine 1. The monitoring apparatus 2 comprises a housing 9 having a sample inlet 10 (see Figure 2) for receiving a sample of metalworking fluid there through from the metalworking fluid circuit 3 and an outlet 11 for discharging the sample of metalworking fluid to the metalworking fluid circuit 3 after measurement. The inlet 10 and the outlet 11 may also be combined into a single connection between the apparatus 2 and the circuit 3. The inlet 10 and the outlet 11 may comprise an openable and closable valve configured to let a sample of metalworking fluid into the monitoring apparatus 2 only when desired. The housing 9 can be configured to engage with a metalworking machine 1, such that, when engaged, the sample inlet 10 is connectable to the metalworking fluid circuit 3. The housing 9 can be provided with engagement elements 12, for example a transverse protruding sheet and fixation elements, for engagement to the metalworking machine 1. The housing 9, in particular the engagement elements 12, can advantageously be configured to fit to a receiving seat 7 of the metalworking machine 1. When the monitoring apparatus 2 is for example configured as an add-on module for a metalworking machine 1, the housing 9 can advantageously be fixated on the metalworking machine 1, an existing or newly built metalworking machine, in said receiving seats 7 using fixation elements 8, for example screws or bolts or any other suitable fixation means. The monitoring apparatus 2 further comprises a water connection 13 for connecting to a water feed line, and a lubricant connection 14 for connecting to a lubricant feed line. The water feed line can for example be connected to a tap water connection point, e.g. of the mains, or to a water tank, the lubricant feed line can for example be connected to a lubricant container.

Figure 2 shows a schematic representation of an inside of the monitoring apparatus 2 of Figure 1. The monitoring apparatus 2 comprises at least one, and preferably a plurality of, measurement elements 15 arranged in the housing 9 for measuring pre-determined parameters of the sample of metalworking fluid. The measurement elements 15 are remote from the metalworking fluid circuit 3, meaning that the measurement elements 15 are not mounted into the fluid circuit 3, as in the prior art devices, and as such, are not permanently exposed to the metalworking fluid. The measurement elements 15 can for example comprise at least one, or preferably more, of a pH-measurement element, a conductivity measurement element, a dissolved oxygen measurement element, a refractometer element, temperature measurement element, and/or a fluid level measurement element, or any other suitable and useful measurement element. The monitoring apparatus 2 can further comprise a measurement chamber 16 into which the at least one measurement element 15 extends. In case of a plurality of measurement elements 15, all measurement elements 15 may be present in, or extend into, the measurement chamber 16. Alternatively, for each measurement element 15, a separate measurement chamber 16 can be provided, or alternatively, a plurality of measurement chambers 16 can be provided, in each measurement chamber one or more measurement elements may extend. A combination of these embodiments is possible as well. The measurement chamber 16 is configured to temporarily receive a sample of metalworking fluid from the metalworking fluid circuit 3, for example via the sample inlet 10 which is connectable to the metalworking fluid circuit 3. By arranging a measurement chamber and at least one measurement element extending in a measurement chamber to measure a sample of metalworking fluid in a housing, a compact device can be provided that is self-contained, meaning that the components needed to perform a measurement run of the sample of fluid are available in the housing. As such, the device can be understood to be modular and may fit onto existing as well as newly-built machines.

After a measurement run, the metalworking fluid can leave the measurement chamber 16 via the outlet 11 for discharging the sample of metalworking fluid to the metalworking fluid circuit 3. A measurement run can be done at predetermined time intervals and/or the time interval between subsequent measurement runs may depend on the condition of the metalworking fluid, as monitored by the monitoring apparatus 2. Also, a measurement run may be performed after an "event", such as the filling of the circuit and/or supplying additional water and/or lubricant to the circuit, has taken place.

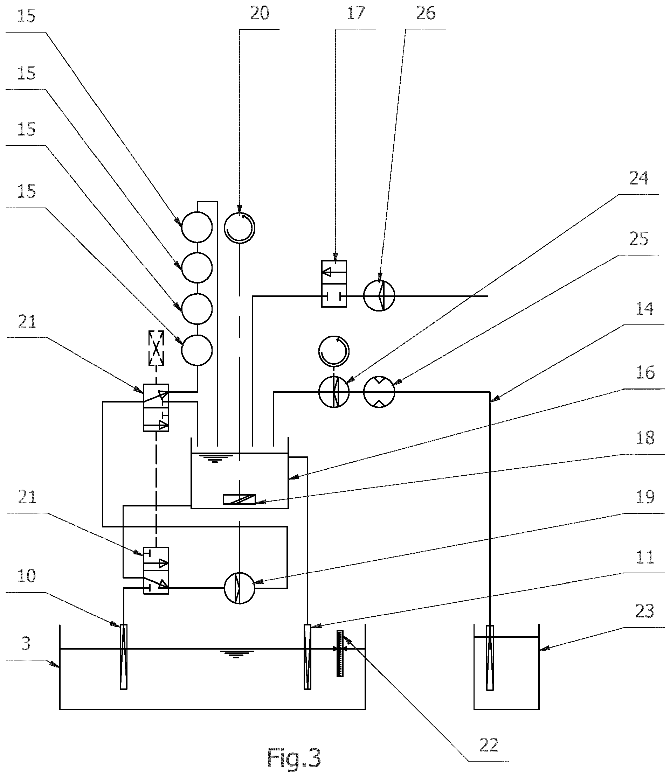

Preferably, after each measurement run, the measurement chamber 16 and/or the measurement elements 15 are rinsed with water for cleaning to prevent contamination and continued exposure of these elements 15 to (residues of) the metalworking fluid. Thereto, a clean water valve 17 may be provided within the housing 9. The monitoring apparatus 2 can also be provided with a mixer 18 extending into the measurement chamber 16, to mix a sample of metalworking fluid prior to a measurement run, as to undo some effects that may have occurred during the flow of the sample of fluid from the fluid circuit 3 to the measurement chamber 16. Alternatively, or additionally, when water and/or lubricant may have to be added to the fluid circuit 3, the determined amount of water and/or lubricant can preferably be entered into the measurement chamber 16 first, for example via the water connection 13 connectable to a water feed line, and/or via a lubricant connection 14 which is connectable to a lubricant feed line, to be mixed or stirred by the mixer 18 prior to being discharged to the metalworking fluid circuit 3. So, preferably, the added metalworking fluid is already mixed when joining the metalworking fluid circuit 3, providing for a more optimal mixture with the metalworking fluid already present in the circuit. The monitoring apparatus 2 can further comprise a pump unit 19 (see Figure 3) arranged in the housing 9 for pumping a sample of the metalworking fluid from the metalworking fluid circuit 3 to the measurement elements 15. The motor 20 of the pump unit 19 can be different from, or the same as, the motor 20 of the mixer 18. Advantageously, the measurement elements 15 are configured to provide the values of the parameters that are measured to a control unit (not shown). The control unit may be integrated to the apparatus 2, i.e. may be arranged in the housing 9 of the apparatus 2. Alternatively, the control unit may be remotely located from the apparatus 2, for example on a remote server, or on a mobile communications device, etc. Many variants are possible. The monitoring apparatus 2 may also include a user feedback element (not shown) that is activated by an output signal of a control unit, when a measured parameter value is outside of a predetermined boundary. The user feedback element can for example comprise a warning light, emitting a light signal, to visually warn a user or an operator, or may be a sound emitter for emitting sound waves to audibly warn a user or an operator. The user feedback element may also be a user interface provided e.g. on the housing of the apparatus on which the information about the respective parameter can be displayed. The output signal of the control unit may also be transmitted to a mobile communication device, or to a control room, or to a remotely located computer etc. The user feedback element may be integrated in an application for a mobile communication device 'app', or a (remote) computer. Many variants known to the person skilled in the art are possible. By providing such an output signal, the operator can be alarmed that one or more parameters have measured values outside of a predetermined boundary

Figure 3 shows a schematic flow diagram of a method for monitoring a condition of metalworking fluid of a metalworking fluid circuit of a metalworking machine. The method includes at least the steps of sampling an amount of metalworking fluid from the metalworking fluid circuit 3, measuring pre-determined parameters on the sample of metalworking fluid, and discharging the sample of metalworking fluid to the metalworking fluid circuit 3. Thereto, the apparatus can comprise a pump unit 19, for example a centrifugal pump driven by a motor 20, for pumping up a sample of the metalworking fluid from the metalworking fluid circuit 3 via the inlet 10. In a preferred embodiment, the apparatus comprises a measurement chamber 16 into which a sample of metalworking fluid can be pumped, and into which the at least one measurement element 15 extends. Before carrying out a measurement on the sample, the sample can preferably be mixed by the mixer 18 to obtain a better mixture of the sample. Measurement of predetermined values can for example include a pH-measurement, a conductivity measurement, a dissolved oxygen measurement, a temperature measurement, or other measurements. It can for example be determined whether the measured parameter values are within pre-determined boundaries, which can for example be stored in a control unit included in the apparatus or located remotely from the apparatus. When at least one of a measured parameter value is outside the pre-determined boundaries, an output signal can be provided, for example to an operator of the metalworking machine. After finishing the measurements, the sample of metalworking fluid can be released back into the metalworking fluid circuit 3 via the outlet 11. This operation can be done at regular time intervals, or when it is deemed necessary. Measurement elements can preferably be rinsed with water after each sample run. A person skilled in the art will understand that one or more openable and closable valves 21 can be included in the apparatus, for example between the inlet 10 and the measurement chamber 16, and/or between the measurement chamber 16 and the at least one measurement element 15, so that the measurement elements 15 are only exposed to the metalworking fluid during measurements.

In a preferred embodiment, the method can also include an improved way of surveying and filling up the metalworking fluid circuit 3. Instead of observing by eye whether the amount of metalworking fluid is enough or not, the monitoring apparatus is preferably provided with a floater 22 configured to indicate when a level of metalworking fluid in the metalworking fluid circuit 3 descends under a predetermined level. Determining the amount or level of metalworking fluid can for example be done at specified time intervals. Instead of directly filling up the circuit 3 with metalworking fluid as such, it is preferred that the concentration of a sample of the metalworking fluid, for example in the measurement chamber 16, is determined or monitored, for example by measuring the refractory index of a sample of metalworking fluid in the measurement chamber 16, preferably by means of a refractometer. On the basis of the determination of the concentration, for example after having calibrated a refractory index of the metalworking fluid, preferably by means of a refractometer measurement element, it can be determined what amount of water and/or what amount of lubricant fluid needs to be supplied until the concentration and/or the fluid level of the metalworking fluid is within a predetermined boundary. The control unit may then control the supply of the determined amount of water and/or lubricant fluid, for example by controlling water feed line 13 and/or the lubricant feed line 14. Alternatively and/or additionally the amount of water and/or lubricant can for example be transmitted in an output signal to a user feedback element. In order to add the needed amount of water and/or lubricant, the apparatus can comprise a lubricant reservoir 23 connected via the lubricant feed line to the lubricant connection 14. The lubricant feed line can also be provided with a lubricant pump 24 and/or a lubricant flow detection device 25 to detect an amount of lubricant being pumped up by the lubricant pump 24 from the lubricant reservoir 23 to the measurement chamber 16. Analogously, the water feed line may be provided with a water flow detection device 26 to detect an amount of water to be supplied to the measurement chamber 16. Preferably, the valve 17 and/or pump 24 are controlled by the control unit receiving feedback from the detection devices 26 and/or 25. Alternatively, controlling the valves may be done manually by the operator. After supplying the additional amount of water and/or lubricant fluid to the measurement chamber 16, the concentration of the metalworking fluid can be determined again, with or without first mixing the metalworking fluid in the measurement chamber 16. In an alternative, more sophisticated, method, the determination of the amount of water and/or lubricant fluid to be supplied can additionally depend on a feedback of a previous water and/or lubricant supply run. So, a learning control and/or feedback loop can be obtained and the control unit can be a learning controller. When a desired concentration of the metalworking fluid in the measurement chamber 16 has been reached, the metalworking fluid is supplied to the metalworking fluid circuit 3.

The method according to an aspect of the invention can be highly automated, for example by adding a control unit (not shown) configured for monitoring a condition of metalworking fluid of a metalworking circuit of a metalworking machine. The control unit may be part of the housing 9 of the monitoring apparatus 2, or may be a separate unit installed remotely from the monitoring apparatus 2. The control unit is configured for receiving values of measured parameters from measurement elements 15 of the monitoring apparatus 2, and can further be configured for determining whether the measured parameter values are within predetermined boundaries. The control unit can also be configured for providing an output signal when at least one of the measured parameter values is outside of a predetermined boundary, preferably to a user feedback element, for example a display, which can also be incorporated into the housing 9 of the monitoring apparatus 2, or which can be placed at a distance from the monitoring apparatus 2. The control unit can further be configured to determine an amount of water and/or an amount of lubricant to be filled when the measured concentration parameter value of the metalworking fluid is outside a predetermined boundary. The control unit can also comprise a computer unit or a mobile communication device, on which a computer program product can be installed for monitoring a condition of a metalworking fluid of a metalworking circuit of a metalworking machine, which computer program product comprises instructions for causing a processor to perform at least one of the steps of the method according to the method as described above.

For the purpose of clarity and a concise description, features are described herein as part of the same or separate embodiments, however, it will be appreciated that the scope of the invention may include embodiments having combinations of all or some of the features described. It may be understood that the embodiments shown have the same or similar components, apart from where they are described as being different.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to an advantage.

The scope of the invention is defined by the appended claims.

## Claims

1. Monitoring apparatus (2) for monitoring a condition of an aqueous
metalworking fluid comprised of water and a lubricant, of a metalworking fluid circuit (3) of a metalworking machine (1), comprising
- a housing (9) having a sample inlet (10) for receiving a sample of metalworking fluid there through from the metalworking fluid circuit;
- an outlet (11) for discharging the sample of metalworking fluid after measurement to the metalworking fluid circuit;
- wherein the housing (9) is provided with a water connection (13) for connecting to a water feed line;
- wherein the housing is provided with a lubricant connection (14) for connecting to a lubricant feed line; comprising at least one measurement element (15) arranged in the housing (9) for measuring pre-determined parameters of the sample of metalworking fluid.

2. Apparatus according to claim 1, further comprising a measurement chamber (16) into which the at least one measurement element (15) extends.

3. Apparatus according to claim 1 or 2, wherein a mixer (18) extending into the measurement chamber (16) is provided.

4. Apparatus according to any one of the preceding claims, further comprising a pump unit (19) arranged in the housing (9) for pumping a sample of
the metalworking fluid from the metalworking fluid circuit (3) to the measurement elements (15).

5. Apparatus according to any one of the preceding claims, wherein the measurement elements (15) are configured for providing the measured parameter values to a control unit.

6. Apparatus according to any one of the preceding claims, wherein the measurement elements (15) comprise at least one of a pH-measurement element, a conductivity measurement element, a dissolved oxygen measurement element, a refractometer element, temperature measurement element, a fluid level measurement element.

7. Apparatus according to any one of the preceding claims, further comprising a user feedback element that is activated by an output signal of a control unit, when a measured parameter value is outside of a predetermined boundary.

8. Apparatus according to any one of the preceding claims, wherein the housing (9) is configured to engage with a metalworking machine (1), such that, when engaged, the sample inlet (10) is connectable to the metalworking fluid circuit (3).

9. Apparatus according to claim 8, wherein the housing (9) is provided with engagement elements (12) for engagement to the metalworking machine (1).

10. Apparatus according to any one of the preceding claims, wherein the housing (9) is configured to fit to a receiving seat (7) of the metalworking machine (1).

11. Apparatus according to any one of the preceding claims, wherein the apparatus (2) is an add-on module for a metalworking machine (1).

12. Control unit configured for monitoring a condition of metalworking fluid of a metalworking circuit (3) of a metalworking machine (1), wherein the control unit is configured for receiving values of measured parameters from measurement elements (15) of an apparatus (2) according to any one of the claims 1 - 11, further is configured for determining whether the measured parameter values are within predetermined boundaries, further configured to determine an amount of water and/or an amount of lubricant to be filled when the measured parameter value of the metal working fluid is outside a predetermined boundary.

13. Control unit according to claim 12, wherein the control unit is configured for providing an output signal when at least one of the measured parameter values is outside of a predetermined boundary, preferably to a user feedback element.

14. System of an apparatus and a control unit, comprising an apparatus (2) according to any one of the claims 1 - 11 and a control unit according to any one of the claims 12 - 13, wherein the control unit is arranged in the housing (9) of the apparatus (2).

15. Method for monitoring a condition of metalworking fluid of a metalworking fluid circuit (3) of a metalworking machine (1) using an apparatus (2) according to any of the claims 1 - 11; comprising the steps of
- sampling an amount of metalworking fluid from the metalworking fluid circuit (3);
- measuring pre-determined parameters on the sample of metalworking fluid;
- discharging the sample of metalworking fluid to the metalworking fluid circuit (3).

16. Method according to claim 15, further comprising determining whether the measured parameter values are within pre-determined boundaries.

17. Method according to claim 16, providing an output signal when at least one of a measured parameter value is outside the pre-determined boundaries.

18. Method according to any of the claims 15 - 17, further comprising determining an amount of water and/or an amount of lubricant fluid to be supplied until the concentration and/or the fluid level of the metalworking fluid is within a predetermined boundary.

19. Method according to claim 18, comprising determining the concentration of the metalworking fluid after supplying of the additional amount of water and/or lubricant fluid.

20. Method according to any one of claims 18 - 19, wherein determining the amount of water and/or lubricant fluid to be supplied additionally depends on a feedback of a previous water and/or lubricant supply run.

21. Method according to any one of the claims 15 - 20, comprising the step of rinsing measurement elements (15) with water after each sample run.

22. Method for determining a condition of a metalworking fluid of a metalworking fluid circuit (3) of a metalworking machine (1) comprising
- receiving measured parameter values from measurement elements (15) of an apparatus (2) according to any of the claims 1 - 11;
- determining whether the measured parameter values are within predetermined boundary;
- outputting an output signal when the measured parameter values are outside of the predetermined boundary.

23. 24.Method according to claim 22, wherein the output signal comprises a control signal to a user feedback element and/or a control signal comprising a determined amount of water and/or a determined amount of lubrication fluid to be supplied until a concentration and/or a fluid level is within a predetermined boundary.

24. Method for retro-fitting a metalworking machine (1) having an individual metalworking fluid circuit (3) comprising
- providing an apparatus (2) according to any one of the claims 1 - 11;
- mounting the apparatus (2) to the metalworking machine, such that the sample inlet (10) is connected to the metalworking fluid circuit (3);
- connecting the apparatus (2) to a water feed line and a lubricant feed line.

25. Computer program product for monitoring a condition of a metalworking fluid of a metalworking circuit of a metalworking machine, which computer program product comprises instructions for causing a processor to perform the steps of the method according to claim 22 or 23.

## Patentansprüche

1. Überwachungseinrichtung (2) zur Überwachung eines Zustands eines aus Wasser und einem Schmiermittel bestehenden wässrigen Kühlschmierstoffs eines Kühlschmierstoffkreislaufs (3) einer Metallbearbeitungsmaschine (1), umfassend
- ein Gehäuse (9) mit einem Probeneinlass (10) zur Aufnahme dadurch einer Kühlschmierstoffprobe von dem Kühlschmierstoffkreislauf;
- einen Auslass (11) zum Ablassen der Kühlschmierstoffprobe nach der Messung in den Kühlschmierstoffkreislauf;
- wobei das Gehäuse (9) mit einem Wasseranschluss (13) zum Anschließen an eine Wasserzuleitung versehen ist;
- wobei das Gehäuse mit einem Schmierstoffanschluss (14) zum Anschließen an eine Schmierstoffzuleitung versehen ist; umfassend wenigstens ein im Gehäuse (9) angeordnetes Messelement (15) zum Messen vorbestimmter Parameter der Kühlschmierstoffprobe.

2. Einrichtung nach Anspruch 1, ferner umfassend eine Messkammer (16), in die sich das wenigstens eine Messelement (15) erstreckt.

3. Einrichtung nach Anspruch 1 oder 2, wobei ein sich in die Messkammer (16) erstreckender Mischer (18) bereitgestellt ist.

4. Einrichtung nach einem der vorhergehenden Ansprüche, ferner umfassend eine im Gehäuse (9) angeordnete Pumpeinheit (19) zum Pumpen einer Probe des Kühlschmierstoffs aus dem Kühlschmierstoffkreislauf (3) zu den Messelementen (15).

5. Einrichtung nach einem der vorhergehenden Ansprüche, wobei die Messelemente (15) zum Bereitstellen der gemessenen Parameterwerte an eine Steuereinheit ausgelegt sind.

6. Einrichtung nach einem der vorhergehenden Ansprüche, wobei die Messelemente (15) wenigstens ein pH-Messelement, ein Leitfähigkeitsmesselement, ein Element zur Messung von gelöstem Sauerstoff, ein Refraktometerelement, ein Temperaturmesselement, ein Flüssigkeitsstandmesselement umfassen.

7. Einrichtung nach einem der vorhergehenden Ansprüche, ferner umfassend ein Benutzerrückkopplungselement, das durch ein Ausgangssignal einer Steuereinheit aktiviert wird, wenn ein gemessener Parameterwert außerhalb einer vorbestimmten Grenze liegt.

8. Einrichtung nach einem der vorhergehenden Ansprüche, wobei das Gehäuse (9) dazu eingerichtet ist, in eine Metallbearbeitungsmaschine (1) einzugreifen, so dass, wenn es eingreift, der Probeneinlass (10) mit dem Kühlschmierstoffkreislauf (3) verbunden werden kann.

9. Einrichtung nach Anspruch 8, wobei das Gehäuse (9) mit Eingriffselementen (12) zum Eingriff in die Metallbearbeitungsmaschine (1) versehen ist.

10. Einrichtung nach einem der vorhergehenden Ansprüche, wobei das Gehäuse (9) so ausgelegt ist, dass es in einen Aufnahmesitz (7) der Metallbearbeitungsmaschine (1) hineinpasst.

11. Einrichtung nach einem der vorhergehenden Ansprüche, wobei die Einrichtung (2) ein Zusatzmodul für eine Metallbearbeitungsmaschine (1) ist.

12. Steuereinheit zur Überwachung eines Zustands eines Kühlschmierstoffs eines Metallbearbeitungskreislaufs (3) einer Metallbearbeitungsmaschine (1), wobei die Steuereinheit zum Empfangen von Werten gemessener Parameter von Messelementen (15) einer Einrichtung (2) nach einem der Ansprüche 1 bis 11 ausgelegt ist, ferner zum Bestimmen ausgelegt ist, ob die gemessenen Parameterwerte innerhalb vorbestimmter Grenzen liegen, ferner so ausgelegt ist, dass sie eine Menge an Wasser und/oder einer Menge an Schmiermittel, die zu füllen ist, bestimmt, wenn der gemessene Parameterwert des Kühlschmierstoffs außerhalb einer vorbestimmten Grenze liegt.

13. Steuereinheit nach Anspruch 12, wobei die Steuereinheit zum Bereitstellen eines Ausgangssignals ausgelegt ist, wenn wenigstens einer der gemessenen Parameterwerte außerhalb einer vorbestimmten Grenze liegt, vorzugsweise an ein Benutzerrückkopplungselement.

14. System einer Einrichtung und einer Steuereinheit, umfassend eine Einrichtung (2) nach einem der Ansprüche 1 bis 11 und eine Steuereinheit nach einem der Ansprüche 12 bis 13, wobei die Steuereinheit im Gehäuse (9) der Einrichtung (2) angeordnet ist.

15. Verfahren zur Überwachung eines Zustands eines Kühlschmierstoffs eines Kühlschmierstoffkreislaufs (3) einer Metallbearbeitungsmaschine (1) unter Verwendung einer Einrichtung (2) nach einem der Ansprüche 1 bis 11; umfassend die Schritte
- Entnahme einer Menge an Kühlschmierstoff aus dem Kühlschmierstoffkreislauf (3);
- Messen vorbestimmter Parameter an der Kühlschmierstoffprobe;
- Ablassen der Kühlschmierstoffprobe in den Kühlschmierstoffkreislauf (3).

16. Verfahren nach Anspruch 15, ferner umfassend das Bestimmen, ob die gemessenen Parameterwerte innerhalb vorbestimmter Grenzen liegen.

17. Verfahren nach Anspruch 16 zum Bereitstellen eines Ausgangssignals, wenn wenigstens einer der gemessenen Parameterwerte außerhalb der vorbestimmten Grenzen liegt.

18. Verfahren nach einem der Ansprüche 15 bis 17, ferner umfassend das Bestimmen einer Menge an Wasser und/oder einer zuzuführenden Menge an flüssigem Schmiermittel, bis die Konzentration und/oder der Flüssigkeitsstand des Kühlschmierstoffs innerhalb einer vorbestimmten Grenze liegt.

19. Verfahren nach Anspruch 18, umfassend das Bestimmen der Konzentration des Kühlschmierstoffs nach dem Zuführen der zusätzlichen Menge an Wasser und/oder flüssigem Schmiermittel.

20. Verfahren nach einem der Ansprüche 18 bis 19, wobei das Bestimmen der zuzuführenden Menge an Wasser und/oder flüssigem Schmiermittel zusätzlich von einer Rückkopplung eines vorangegangenen Wasser- und/oder Schmiermittelzuführungsdurchlaufs abhängt.

21. Verfahren nach einem der Ansprüche 15 bis 20, umfassend den 30 Schritt des Spülens von Messelementen (15) mit Wasser nach jedem Probendurchlauf.

22. Verfahren zum Bestimmen des Zustands eines Kühlschmierstoffs eines Kühlschmierstoffkreislaufs (3) einer Metallbearbeitungsmaschine (1) umfassend
- Empfangen von gemessenen Parameterwerten von Messelementen (15) einer Einrichtung (2) nach einem der Ansprüche 1 bis 11;
- Bestimmen, ob die gemessenen Parameterwerte innerhalb der vorbestimmten Grenzen liegen;
- Ausgabe eines Ausgangssignals, wenn die gemessenen Parameterwerte außerhalb der vorbestimmten Grenze liegen.

23. Verfahren nach Anspruch 22, wobei das Ausgangssignal ein Steuersignal an ein Benutzerrückkopplungselement und/oder ein Steuersignal umfasst, das eine bestimmte Menge an Wasser und/oder eine bestimmte Menge an zuzuführendem flüssigem Schmiermittel umfasst, bis eine Konzentration und/oder ein Flüssigkeitsstand innerhalb einer vorbestimmten Grenze liegt.

24. Verfahren zur Nachrüstung einer Metallbearbeitungsmaschine (1) mit einem individuellen Kühlschmierstoffkreislauf (3) umfassend
- Bereitstellen einer Einrichtung (2) nach einem der Ansprüche 1 bis 11;
- Montieren der Einrichtung (2) an der Metallbearbeitungsmaschine, so dass der Probeneinlass (10) mit dem Kühlschmierstoffkreislauf (3) verbunden ist;
- Anschließen der Einrichtung (2) an eine Wasserzuleitung und eine Schmierstoffzuleitung.

25. Computerprogrammprodukt zur Überwachung eines Zustands eines Kühlschmierstoffs eines Metallbearbeitungskreislaufs einer Metallbearbeitungsmaschine, wobei das Computerprogrammprodukt Anweisungen umfasst, um zu bewirken, dass ein Prozessor die Schritte des Verfahrens nach Anspruch 22 oder 23 ausführt.

## Revendications

1. Appareil de surveillance (2) pour surveiller l'état d'un fluide de travail des métaux aqueux composé d'eau et d'un lubrifiant, d'un circuit de fluide de travail des métaux (3) d'une machine de travail des métaux (1), comprenant :
- un boîtier (9) ayant une entrée d'échantillon (10) pour recevoir un échantillon de fluide de travail des métaux à travers celui-ci provenant du circuit de fluide de travail des métaux ;
- une sortie (11) pour évacuer l'échantillon de fluide de travail des métaux après mesure vers le circuit de fluide de travail des métaux ;
- dans lequel le boîtier (9) est pourvu d'un raccord d'eau (13) pour le raccordement à une conduite d'alimentation en eau ;
- dans lequel le boîtier est pourvu d'un raccord de lubrifiant (14) pour se connecter à une conduite d'alimentation en lubrifiant ; comprenant au moins un élément de mesure (15) agencé dans le boîtier (9) pour mesurer des paramètres prédéterminés de l'échantillon de fluide de travail des métaux.

2. Appareil selon la revendication 1, comprenant en outre une chambre de mesure (16) dans laquelle s'étend le au moins un élément de mesure (15).

3. Appareil selon la revendication 1 ou 2, dans lequel un mélangeur (18) s'étendant dans la chambre de mesure (16) est prévu.

4. Appareil selon l'une quelconque des revendications précédentes, comprenant en outre une unité de pompe (19) agencée dans le boîtier (9) pour pomper un échantillon du fluide de travail des métaux du circuit de fluide de travail des métaux (3) vers les éléments de mesure (15).

5. Appareil selon l'une quelconque des revendications précédentes, dans lequel les éléments de mesure (15) sont configurés pour fournir les valeurs de paramètre mesurées à une unité de commande.

6. Appareil selon l'une quelconque des revendications précédentes, dans lequel les éléments de mesure (15) comprennent au moins l'un parmi un élément de mesure du pH, un élément de mesure de la conductivité, un élément de mesure de l'oxygène dissous, un élément formant réfractomètre, un élément de mesure de la température, un élément de mesure du niveau de fluide.

7. Appareil selon l'une quelconque des revendications précédentes, comprenant en outre un élément de rétroaction utilisateur qui est activé par un signal de sortie d'une unité de commande, lorsqu'une valeur de paramètre mesurée est en dehors d'une limite prédéterminée.

8. Appareil selon l'une quelconque des revendications précédentes, dans lequel le boîtier (9) est configuré pour coopérer avec une machine à travailler les métaux (1), de sorte que, lorsqu'il est engagé, l'entrée d'échantillon (10) est connectable au circuit de fluide de travail des métaux (3).

9. Appareil selon la revendication 8, dans lequel le boîtier (9) est pourvu d'éléments d'engagement (12) pour l'engagement avec la machine à travailler les métaux (1).

10. Appareil selon l'une quelconque des revendications précédentes, dans lequel le boîtier (9) est configuré pour s'adapter à un siège de réception (7) de la machine à travailler les métaux (1).

11. Appareil selon l'une quelconque des revendications précédentes, dans lequel l'appareil (2) est un module complémentaire pour une machine à travailler les métaux (1).

12. Unité de commande configurée pour surveiller un état du fluide de travail des métaux d'un circuit de travail des métaux (3) d'une machine à travailler les métaux (1), dans laquelle l'unité de commande est configurée pour recevoir des valeurs de paramètre mesurées à partir d'éléments de mesure (15) d'un appareil (2) selon l'une quelconque des revendications 1 à 11, est en outre configuré pour déterminer si les valeurs de paramètre mesurée sont dans des limites prédéterminées, configurées en outre pour déterminer une quantité d'eau et/ou une quantité de lubrifiant à remplir lorsque la valeur de paramètre mesurée du fluide de travail des métaux est en dehors d'une limite prédéterminée.

13. Unité de commande selon la revendication 12, dans laquelle l'unité de commande est configurée pour fournir un signal de sortie lorsqu'au moins une des valeurs de paramètre mesurées est en dehors d'une limite prédéterminée, de préférence à un élément de rétroaction utilisateur.

14. Système composé d'un appareil et d'une unité de commande, comprenant un appareil (2) selon l'une quelconque des revendications 1 à 11 et une unité de commande selon l'une quelconque des revendications 12 à 13, dans lequel l'unité de commande est agencée dans le boîtier (9) de l'appareil (2).

15. Procédé de surveillance d'un état de fluide de travail des métaux d'un circuit de fluide de travail des métaux (3) d'une machine à travailler les métaux (1) utilisant un appareil (2) selon l'une quelconque des revendications 1 à 11 ; comprenant les étapes consistant à :
- prélever une quantité de fluide de travail des métaux à partir du circuit de fluide de travail des métaux (3) ;
- mesurer des paramètres prédéterminés sur l'échantillon de fluide de travail des métaux ;
- décharger l'échantillon de fluide de travail des métaux dans le circuit de fluide de travail des métaux (3).

16. Procédé selon la revendication 15, comprenant en outre la détermination du fait que les valeurs de paramètre mesurées sont dans des limites prédéterminées.

17. Procédé selon la revendication 16, fournissant un signal de sortie lorsqu'au moins l'une d'une valeur de paramètre mesurée est en dehors des limites prédéterminées.

18. Procédé selon l'une quelconque des revendications 15 à 17, comprenant en outre la détermination d'une quantité d'eau et/ou d'une quantité de fluide lubrifiant à fournir jusqu'à ce que la concentration et/ou le niveau de fluide du fluide de travail des métaux soit dans une limite prédéterminée.

19. Procédé selon la revendication 18, comprenant la détermination de la concentration du fluide de travail des métaux après apport du complément quantité d'eau et/ou de fluide lubrifiant.

20. Procédé selon l'une quelconque des revendications 18 à 19, dans lequel la détermination de la quantité d'eau et/ou de fluide lubrifiant à fournir dépend en outre d'un retour d'un cycle d'alimentation en eau et/ou lubrifiant précédent.

21. Procédé selon l'une quelconque des revendications 15 à 20 comprenant l'étape de rinçage des éléments de mesure (15) avec de l'eau après chaque passage d'échantillon.

22. Procédé de détermination d'un état d'un fluide de travail des métaux d'un circuit de fluide de travail des métaux (3) d'une machine de travail des métaux (1) comprenant les étapes consistant à :
- recevoir des valeurs de paramètre mesurées à partir d'éléments de mesure (15) d'un appareil (2) selon l'une quelconque des revendications 1 à 11 ;
- déterminer si les valeurs de paramètre mesurées sont dans une limite prédéterminée ;
- délivrer un signal de sortie lorsque les valeurs de paramètre mesurées sont à en dehors de la limite prédéterminée.

23. Procédé selon la revendication 22, dans lequel le signal de sortie comprend un signal de commande à un élément de rétroaction utilisateur et/ou un signal de commande comprenant une quantité déterminée d'eau et/ou une quantité déterminée de fluide de lubrification à fournir jusqu'à ce qu'une concentration et/ou un niveau de fluide soit dans une limite prédéterminée.

24. Procédé de rétro-équipement d'une machine à travailler les métaux (1) ayant un circuit individuel de fluide de travail des métaux (3) comprenant les étapes consistant à :
- fournir un appareil (2) selon l'une quelconque des revendications 1 à 11
- monter l'appareil (2) sur la machine à travailler les métaux, de sorte que l'entrée d'échantillon (10) soit reliée au circuit de fluide de travail des métaux (3) :
- raccorder l'appareil (2) à une conduite d'alimentation en eau et à une conduite d'alimentation en lubrifiant.

25. Produit formant programme informatique pour surveiller un état d'un fluide de travail des métaux d'un circuit de travail des métaux d'une machine à travailler les métaux, lequel produit formant programme informatique comprenant des instructions pour amener un processeur à exécuter les étapes du procédé selon la revendication 22 ou 23.
